Europäisches Patentamt

**European Patent Office**

**Office européen des brevets**

(11) Veröffentlichungsnummer: **0 044 437**
**B1**

(12) **EUROPÄISCHE PATENTSCHRIFT**

(45) Veröffentlichungstag der Patentschrift:
**15.02.84**

(51) Int. Cl.³: **C 01 B 31/08,** C 07 C 69/44,
C 07 C 69/80

(21) Anmeldenummer: **81104976.6**

(22) Anmeldetag: **26.06.81**

(54) **Verfahren zur Reaktivierung von Aktivkohle.**

(30) Priorität: **17.07.80 AT 3704/80**

(43) Veröffentlichungstag der Anmeldung:
**27.01.82 Patentblatt 82/4**

(45) Bekanntmachung des Hinweises auf die Patenterteilung:
**15.02.84 Patentblatt 84/7**

(84) Benannte Vertragsstaaten:
**AT DE FR GB IT NL**

(56) Entgegenhaltungen:
**CHEMICAL ABSTRACTS, Band 91, Heft 12, 17.
September 1979, Seite 284, Zusammenfassung 95973k,
COLUMBUS, OHIO (US)
JOURNAL OF APPLIED CHEMISTRY OF USSR, Band 52,
Heft 11, November 1979, Teil 2, PLENUM PUBL. CORP.
NEW YORK (US) N. Ya. YAKUBENYA et al.:
"Reactivation of active carbon by removal of
high-boiling organic substances" Seiten 2404-2408**

(73) Patentinhaber: **CHEMIE LINZ AKTIENGESELLSCHAFT,
St. Peter-Strasse 25, A-4020 Linz (AT)**

(84) Benannte Vertragsstaaten: **FR GB IT NL AT**

(73) Patentinhaber: **Lentia Gesellschaft mit beschränkter
Haftung, Arabellastrasse 4 Postfach 81 05 08,
D-8000 München 81 (DE)**

(84) Benannte Vertragsstaaten: **DE**

(72) Erfinder: **Deutner, Wolfgang, Dipl.-Ing., Voltastrasse 69,
A-4045 Linz (AT)**
Erfinder: **Stadlmann, Walter, Dr., Voltastrasse 79,
A-4045 Linz (AT)**
Erfinder: **Kunsch, Andreas, Dipl.-Ing.,
Nöbauerstrasse 36, A-4043 Linz (AT)**
Erfinder: **Weigl, Erich, Sommerstrasse 16, A-4020 Linz
(AT)**
Erfinder: **Schweller, Helmut, Hittmalrstrasse 19,
A-4020 Linz (AT)**
Erfinder: **Lechner, Walter, Schumpeterstrasse 2a,
A-4040 Linz (AT)**

Verfahren zur Reaktivierung von Aktivkohle

Die Erfindung betrifft ein Verfahren zur Reaktivierung von Aktivkohle nach ihrer Verwendung bei der Reinigung von als Weichmacher dienenden Dicarbonsäureestern z.B. von Estern der Phthalsäure oder Adipinsäure, die unter Verwendung von Schwefelsäure oder Toluolsulfonsäure als Katalysator hergestellt werden.

Die Verwendung von Schwefelsäure als Katalysator für die Veresterung bei der Weichmacherherstellung ist seit Jahrzehnten bekannt und hat sich auch vielfach bestens bewährt. Dass heute dennoch der überwiegende Teil der Weltproduktion an Weichmacherestern nicht mehr nach dem Schwefelsäureverfahren hergestellt wird, liegt an den dabei entstehenden Nebenprodukten, die zum Teil stark gefärbt sind. Diese gefärbten Nebenprodukte müssen nun mit verhältnismässig grossen Mengen Aktivkohle entfernt werden, da eine möglichst niedrige Farbzahl ein wichtiges Qualitätsmerkmal für Weichmacher darstellt.

Die gebrauchte Aktivkohle wurde bisher verworfen, da nach dem Abfiltrieren der organischen Phase trotz nachfolgender Wasserdampfbehandlung und Benzinextraktion gemeinsam mit der Kohle nicht nur bis zu 30 Gew.% Dicarbonsäureester bezogen auf die Aktivkohle, sondern zusätzlich etwa die doppelte Menge an im Reaktionsgemisch schwer löslicher Salze wie Natriumcarbonat, Natriumsulfat, Natriumphthalat u.a. zurückbleiben. Eine derartig durch anorganische und organische Verbindungen verunreinigte Aktivkohle ist auf wirtschaftliche Weise nicht reaktivierbar.

In Fällen, in denen Aktivkohle zur Adsorption von Verunreinigungen aus wässrigen Lösungen verwendet wird, kann ihre Reaktivierung wesentlich einfacher sein. So ist nach ihrer Verwendung zum Reinigen wässriger Dialkaliterephthalatlösungen ein mehrmaliges, allerdings stundenlanges Waschen mit alkalischen, wässrigen Lösungen bzw. ein mehrmaliges Waschen mit reinem Wasser ausreichend, wobei die ursprüngliche Aktivität aber nicht voll erreicht wird (DT-PS 1 191 359).

Überraschenderweise wurde nun gefunden, dass sich in organischer Phase verwendete Aktivkohle, die bei der Entfärbung von als Weichmacher dienenden Dicarbonsäureestern mit aliphatischen Alkoholen z.B. von Estern der Phthalsäure oder Adipinsäure anfällt, gleichfalls durch Waschen mit Wasser reinigen lässt. Diese Tatsache ist vor allem deswegen besonders überraschend, weil im Gegensatz dazu, die Farbzahl desselben gefärbten Esters durch Waschen mit entsprechenden Mengen Wasser sich nicht ausreichend senken lässt, d.h. dass die gefärbten Verbindungen in der organischen Phase zurückbleiben. Ausserdem bleiben in der Kohle trotz Entfernens des Grossteils des anhaftenden Dicarbonsäureesters durch Ausblasen mit Stickstoff, Extraktion mit Benzin und Behandlung mit Wasserdampf, wie schon erwähnt, erhebliche Estermengen zurück, welche die unerwünschten Farbstoffe erwartungsgemäss zurückhalten sollten. Überraschenderweise sind diese aber doch durch einfaches Waschen mit Wasser zu entfernen, womit die Aktivkohle wieder voll einsatzfähig wird. Dabei bleiben aber die erheblichen Estermengen in der Aktivkohle zurück und gehen somit nicht verloren, ausgewaschen werden nur die anorganischen und organischen Salze, wie Natriumsulfat, Natriumcarbonat und Natriumphthalat u.a. sowie die gefärbten Substanzen.

Gegenstand der Erfindung ist somit ein Verfahren zur Reaktivierung von Aktivkohle, die bei der Herstellung von Dicarbonsäuredialkylestern, insbesondere Phthalsäuredialkylestern oder Adipinsäuredialkylestern, mit Hilfe von Schwefelsäure oder Toluolsulfonsäure als Katalysatoren eingesetzt wird, welches dadurch gekennzeichnet ist, dass diese nach dem Abfiltrieren und Entfernen der Hauptmenge des anhaftenden Dicarbonsäureesters durch Einblasen von Stickstoff, Benzinextraktion und Einblasen von Wasserdampf mit der 10 bis 150 fachen Gewichtsmenge Wasser bezogen auf die Menge der eingesetzten Aktivkohle bei 40 bis 105 °C 1 bis 60 Minuten lang gewaschen wird.

Die eingesetzten Wassermengen betragen bevorzugt das 40 bis 100fache der verbrauchten Aktivkohlenmenge, wobei die bevorzugten Waschzeiten zwischen 3 und 30 Minuten betragen. Sie sind naturgemäss umso kürzer, je höher die Wassertemperaturen sind, die vorzugsweise zwischen 60 und 103 °C liegen.

Ein Waschen im Kreislauf ist für den Reaktivierungsgrad der Aktivkohle nicht nachteilig, sondern vielmehr die rationellste Reinigungsmethode. Nach dem Trocknen ist die Aktivkohle wieder voll einsatzfähig.

Die nachfolgenden Beispiele sollen das erfindungsgemässe Verfahren näher erläutern.

Beispiel 1:

In einer Weichmacheranlage werden nach Beendigung der Herstellung von 16 t 2-Ethylhexylphthalat 40 kg Aktivkohle gemeinsam mit den durch die Neutralisation der konzentrierten Schwefelsäure (48 kg) und der übrigen sauren Bestandteile entstehenden im Ester schwer löslichen anorganischen und organischen Salzen, wie Natriumsulfat, Natriumcarbonat, und Natriumphthalat u.a. sowie mit ca. 210 kg anhaftendem 2-Ethylhexylphthalats, insgesamt also etwa 400 kg, abfiltriert. Um einen Grossteil der Esterreste zu entfernen, wird mit Stickstoff ausgeblasen und mit 3000 l Leichtbenzin extrahiert. Anschliessend wird mit Wasserdampf das Leichtbenzin entfernt. Es bleiben insgesamt 40 kg erschöpfter Aktivkohle zurück, die noch mit etwa 30 Gew.% 2-Ethylhexylphthalat (12 kg) sowie mit ca. 120 kg anorganischen und organischen Salzen sowie Wasser vermengt sind; das sind insgesamt ca. 200 kg. Dieser Filterrückstand wird mit 3 m³ Wasser (Gewichtsverhältnis Aktivkohle zu Wasser etwa 1:75) bei einer Temperatur von 80 °C 10 Minuten im Kreislauf gewaschen und die verbleibende salzfreie, aber esterhaltige Aktivkohle abfiltriert und getrocknet. Diese ist damit wieder voll wirksam.

Beispiel 2:

Nach der Herstellung von 9 to Diisononylphthalat fallen ca. 25 kg erschöpfter Aktivkohle an, die nach dem in Beispiel 1 beschriebenen Entfernen des Grossteils der Reste an diesem Ester noch mit etwa 25 Gew.% Diisononylphthalat (6 kg) und etwa 70 kg anorganischen und organischen Salzen sowie Wasser vermengt sind; das sind insgesamt ca. 120 kg. Dieser Filterrückstand wird mit 3 m³ Wasser von 50 °C 60 Minuten im Kreislauf gewaschen (Gewichtsverhältnis Aktivkohle zu Wasser 1:120) und die verbleibende salzfreie, aber esterhaltige Aktivkohle abfiltriert und getrocknet. Diese ist damit wieder voll wirksam.

Beispiel 3:

Nach der Herstellung von 16 to Diisodezylphthalat fallen ca. 50 kg erschöpfter Aktivkohle an, die nach dem in Beispiel 1 beschriebenen Entfernen des Grossteils der Esterreste noch mit etwa 20 Gew.% Diisodezylphthalat (10 kg) und etwa 110 kg anorganische und organische Salzen sowie Wasser vermengt sind; das sind insgesamt ca. 200 kg. Dieser Filterrückstand wird mit 1,5 m³ Wasser von 103 °C 2 Minuten im Kreislauf gewaschen (Gewichtsverhältnis zu Wasser 1:30) und die verbleibende salzfreie, aber esterhaltige Aktivkohle abfiltriert und getrocknet. Diese ist damit wieder voll wirksam.

## Patentansprüche

1. Verfahren zur Reaktivierung von Aktivkohle, die bei der Herstellung von Dicarbonsäuredialkylestern, insbesondere Phthalsäuredialkylestern oder Adipinsäuredialkylestern, mit Hilfe von Schwefelsäure oder Toluolsulfonsäure als Katalysatoren verwendet wird, dadurch gekennzeichnet, dass diese nach dem Abfiltrieren und Entfernen der Hauptmenge des anhaftenden Dicarbonsäuredialkylesters mittels Einblasen von Stickstoff, Benzinextraktion und anschliessendes Einblasen von Wasserdampf mit der 10 bis 150fachen Gewichtsmenge Wasser bezogen auf die Menge der eingesetzten Aktivkohle bei 40 bis 105 °C bis 60 Minuten lang gewaschen wird.

2. Verfahren nach Anspruch 1, dadurch gekennzeichnet, dass die eingesetzten Wassermengen das 40 bis 100fache der verbrauchten Aktivkohlemenge betragen.

3. Verfahren nach den Ansprüchen 1 und 2, dadurch gekennzeichnet, dass die Waschzeiten 3 bis 30 Minuten betragen.

4. Verfahren nach den Ansprüchen 1 bis 3, dadurch gekennzeichnet, dass die Wassertemperaturen 60 bis 103 °C betragen.

5. Verfahren nach den Ansprüchen 1 bis 4, dadurch gekennzeichnet, dass die Wäsche der verbrauchten Aktivkohle im Kreislauf vorgenommen wird.

## Revendications

1. Procédé pour la réactivation de charbon actif utilisé lors de la production d'esters dialcoyliques d'acides dicarboxyliques, en particulier d'esters dialcoyliques d'acide phtalique ou d'esters dialcoyliques d'acide adipique, à l'aide d'acide sulfurique ou d'acide toluènesulfonique comme catalyseur, caractérisé en ce qu'on lave ce charbon, après séparation par filtration et élimination de la plus grande partie de l'ester dialcoylique d'acide dicarboxylique qui y adhère, par insufflation d'azote, extraction à l'essence, puis insufflation de vapeur d'eau, pendant un laps de temps de 1 à 60 minutes, avec 10 à 150 fois son poids d'eau par rapport à la quantité de charbon actif utilisé, à une température de 40 à 105 °C.

2. Procédé suivant la revendication 1, caractérisé en ce que la quantité d'eau utilisée représente de 40 à 100 fois la quantité de charbon actif employée.

3. Procédé suivant les revendications 1 et 2, caractérisé en ce que les temps de lavage vont de 3 à 30 minutes.

4. Procédé suivant les revendications 1 à 3, caractérisé en ce que les températures de l'eau sont comprises entre 60 et 103 °C.

5. Procédé suivant les revendications 1 à 4, caractérisé en ce que le lavage du charbon actif usé est effectué en circuit fermé.

## Claims

1. Process for reactivating active charcoal which is used in the preparation of dialkyl dicarboxylates, in particular dialkyl phthalates or dialkyl adipates, with the aid of sulphuric acid or toluenesulphonic acid as catalysts, characterised in that, after filtering-off and removal of most of the adhering dialkyl dicarboxylate by blowing in nitrogen, extracting with gasoline and subsequently blowing in steam, the charcoal is washed with 10 to 150 times the amount by weight of water, based on the amount of active charcoal employed, at 40 to 105 °C for 1 to 60 minutes.

2. Process according to Claim 1, characterised in that the amounts of water employed are 40 to 100 times the amount of spent active charcoal.

3. Process according to Claims 1 and 2, characterised in that the washing times are 3 to 30 minutes.

4. Process according to Claims 1 to 3, characterised in that the water temperatures are 60 to 103 °C.

5. Process according to Claims 1 to 4, characterised in that washing of the spent active charcoal is carried out in circulation.